# EUROPEAN PATENT APPLICATION

(11) **EP 4 653 014 A1**
(43) Date of publication of application: **26.11.2025**
(21) Application number: 24744638.8
(22) Date of filing: 16.01.2024
(51) Int. Cl.: A61K 45/00, A61K 31/423, A61P 35/00, A61P 1/16

(54) **BILIARY TRACT CANCER TREATMENT AGENT**

(30) Priority: 16.01.2023 JP 2023004195; 25.04.2023 JP 2023071340
(71) Applicant: J-Pharma Co., Ltd., Tokyo 105-0013 (JP)
(72) Inventor: YOSHITAKE Masuhiro, Tokyo 105-0013 (JP); KUSAKA Haruki, Tokyo 105-0013 (JP)
(74) Representative: TBK
(86) International application number: PCT/JP2024/000901
(87) International publication number: WO 2024/154717

(57) **Abstract**

The present invention provides a treatment agent for the treatment of curatively unresectable biliary tract cancer that has become refractory or intolerant to cancer chemotherapy and has worsened in a subject, comprising an LAT1 inhibitor, wherein said subject is a LAT1-high expressing patient having Non-Rapid N-acetyltransferase 2.

## Description

### Technical Field

The present invention relates to a treatment agent for biliary tract cancer which comprises L-type amino acid transporter 1 (LAT1) inhibitors, particularly O-(5-amino-2-phenylbenzoxazol-7-yl)methyl-3,5-dichloro-L-tyrosine (generic name: Nanvuranlat), or a pharmaceutically acceptable salt thereof, wherein the treatment agent is used for treating curatively unresectable biliary tract cancer with high LAT1 expression with Non-Rapid N-acetyltransferase 2 that has become refractory to cancer chemotherapy and has worsened, and is optimally used to treat certain subtypes of biliary tract cancer.

Furthermore, the present invention relates to a method of treating biliary tract cancer, comprising administering a LAT1 inhibitor, in particular a biliary tract cancer treatment agent containing Nanvuranlat or a pharmaceutically acceptable salt thereof, to a patient of a specific subtype of biliary tract cancer and having curatively unresectable biliary tract cancer with high LAT1 expression with Non-Rapid NAT2 that has become refractory to cancer chemotherapy and has worsened.

### [Background Art]

Biliary tract cancer is a general term for cancer of the biliary tract, which is classified into intrahepatic biliary tract cancer, extrahepatic biliary tract cancer, gall bladder cancer, and papillary duodenal cancer, depending on the site of origin. Extrahepatic biliary tract cancer is further classified into hilar biliary tract cancer and distal biliary tract cancer. Since intrahepatic biliary tract cancer originates from the bile ducts within the liver, biliary tract cancer is often asymptomatic, although it can be detected during medical examinations and other tests when the disease is in its early stages. As the disease progresses, symptoms include jaundice, pain in the right side of the abdomen, and weight loss, a condition that often leads to diagnosis. Biliary tract cancer is characterized by the fact that it is primary in the epithelium of the lumenal wall of the bile duct and easily invades the surrounding blood vessels and lymphatics due to the fragile serous membrane of the tissue, and is generally fast-growing, progressive, and prone to metastasis.

According to the Japanese Society of Hepato-Biliary-Pancreatic Surgery, 7th edition, biliary tract cancer, gallbladder cancer, and duodenal papillary cancer are classified into Stages I-IV, which are determined by a combination of three categories (TNM classification): T (tumor size), N (degree of lymph node involvement), and M (distant metastasis). In the case of bile duct and gall bladder cancers, tumors that remain within the wall are classified as Stages I and II, those that invade the serosa and spread to adjacent organs and lymph nodes are classified as Stage III, and those with further distal metastasis are classified as Stage IV. Based on this staging system, a treatment plan (surgery, drug therapy, or palliative treatment) is determined based on the patient's condition and test results.

According to cancer statistics (National Cancer Registry incidence data and Vital Statistics mortality data) published by the National Cancer Center, the number of people diagnosed with gall bladder and biliary tract cancer in Japan is 22,159 (2019), ranking 16th among all cancer types. On the other hand, the number of deaths is 18,172 (in 2021), ranking 6th overall, and the 5-year relative survival rate (survival rate data from the 2009-2011 Regional Cancer Registries) is low at 24.5%, ranking second only to pancreatic cancer. When clinical progression at diagnosis is classified into primary (confined to the primary organ), regional (metastasis to a regional lymph node or invasion of an adjacent organ), and distant metastasis (metastasis or invasion of a distant organ or lymph node), the 5-year relative survival rate (2009-2011) for gall bladder and biliary tract cancers at the respective progression levels were 61.0%, 28.5%, and 2.9%, while the 5-year relative survival rate for all sites is 18.5% for patients with distant metastases, and so the prognosis for gall bladder and bile duct cancer is extremely poor, ranking second only to pancreatic cancer (1.8%).

In the Hospital-Based Cancer Registry (2020), of the 18,750 registered cases of gall bladder and biliary tract cancers, 88% were 65 years or older and 60% were at 75 years or older at the time of diagnosis, indicating that the patients were diagnosed at an older age. Among the gall bladder cancer cases included in the tally, the percentage of TNM classification overall Stage IV was very high at 45.1%, and the percentage of Stage IV cases not treated with surgery or drug therapy was 42.4%, accounting for nearly half of the cases.

As described above, gallbladder and biliary tract cancers are diseases with an extremely low survival rate because it is diagnosed at a relatively older age at the time of initial diagnosis, and a large percentage of patients are diagnosed with distant metastasis (Stage IV), and treatment options are limited.

The diagnosis of biliary tract cancer is determined according to the revised third edition of the Japanese Society of Hepatobiliary and Pancreatic Surgery practice algorithm¹. It is recommended to perform blood tests and abdominal ultrasonography at the first step, CT(Computed Tomography) and MRI(Magnetic Resonance Imaging) to evaluate the localization and extent of the lesion at the second step, and cytological diagnoses by direct visual biopsy such as ERCP(Endoscopic Retrograde Cholangiopancreatography) / IDUS(Intraductal Ultrasonography) / POCS(Peroral Cholangioscopy) at the third step. In intrahepatic bile duct cancer, atypical cases with heterogeneous early staining are known, and differentiation from hepatocellular carcinoma is important. Therefore, differentiation of histologic types by tumor biopsy is very important in the selection of drug therapy. In any case, early diagnosis of biliary tract cancer is difficult both anatomically and clinically, and many cases (70%) are Stage III/IV at the time of initial diagnosis.

Biliary tract cancer (BTC) is a heterogeneous group of tumors arising from epithelial cells of the intrahepatic bile duct, extrahepatic bile duct, gall bladder, or duodenal papillary region and are called intrahepatic biliary tract cancer (IHC), extrahepatic biliary tract cancer (EHC), gall bladder cancer (GBC), or duodenal papillary region cancer (AVC), respectively. BTC is a malignancy that is very difficult to manage clinically and often becomes resistant to treatment².

On the other hand, N-acetylation polymorphisms were discovered more than 50 years ago, as individual differences in isoniazid neurotoxicity are attributed to genetic differences in N-acetylation capacity. In addition to isoniazid, many aromatic amine drugs such as sulfamethazine are affected by the acetylation polymorphism, affecting the therapeutic efficacy and toxicity of many treatment agents. NAT2 is an enzyme that transfers the acetyl group of acetyl CoA to aromatic amines and hydrazine-containing compounds that are substrates for this enzyme. NAT2 is encoded by the 870-bp gene, and genetic polymorphisms of NAT2 are common in humans.

It is known that there are three types of NAT2: Rapid, Intermediate, and Slow (SNP; single nucleotide polymorphism) ( Pharmacogenomics, vol. 13: pp. 31-41, 2012). These types can be distinguished by their genetic polymorphisms.

Genetic polymorphisms in NAT2 are known to modify both the efficacy and toxicity of a number of arylamine and hydrazine drugs and increase the risk for some arylamine carcinogen-related cancers. It is also known that the drug sulfasalazine (SSZ), which is acetylated by NAT2, is often prone to adverse effects in patients with slow acetylation (J Rheumatol. 2002; Dec;29(12):2492-2499) (Genotyping the NAT2 gene followed by estimation of diplotype configuration before administration of SSZ is likely to reduce the frequency of adverse effects in Japanese patients with RA.http://www.jrheum.org/content/29/12/2492).

Representative genetic polymorphisms of NAT2 are (i) 191G>A (rs1801279), (ii) 282C>T (rs1041983), (iii) 341T>C (rs1801280), (iv) 481C>T (rs1799929), (v) 590G>A (rs1799930), (v (i) 803A>G (rs1208), and (vii) 857G>A (rs1799931) are seven SNPs. Among them, SNPs (i), (iii), (v), (vi) and (vii) result in amino acid changes (i.e., (i) 191G>A is arginine to glutamic acid, (ii) 341T>C is isoleucine to threonine, (v) 590G>A is arginine to glutamine, (vi (803A>G is lysine to arginine, (vii) 857G>A is glycine to glutamic acid).

On the other hand, in tumor cells, the expression of transporters that take in nutrients such as sugars and amino acids from the outside is increased to maintain rapid cell growth and enhanced intracellular metabolism. In particular, LAT1, a transporter specifically expressed in tumor cells, transports essential amino acids including leucine, which is also a signaling factor, and plays an important role in supplying essential nutrients to tumor cells. In contrast, LAT2 (L-type amino acid transporter 2) is known to be widely expressed in normal cells. Therefore, compounds with selective inhibitory activity against LAT1 could be anti-cancer agents with fewer side effects.

LAT1 is responsible for transporting large neutral amino acids across the cancer protoplasmic membrane and plays a pivotal role in energy production³. LAT1 is ubiquitously expressed in all subtypes of BTC⁴, especially in the aggressively proliferating phase⁵. LAT1 is essential for cancer growth and its expression level is hypothesized to be a prognostic marker^{6,7}.

As a compound with selective inhibitory activity against LAT1, O-(5-amino-2-phenylbenzoxazol-7-yl) methyl-3,5-dichloro-L-tyrosine (Nanvuranlat, development code "JPH203") is known.

Nanvuranlat is a selective inhibitor of LAT1. Inhibition of tumor growth has been demonstrated in human BTC-derived cell lines both in vitro and in vivo⁸. Inhibition of LAT1 induces mTORC1 signaling, resulting in extensive downregulation of cancer cell functions such as protein synthesis and cell growth^{9,10}.

A Phase I dose-escalation trial of Nanvuranlat was conducted in 17 patients with a variety of solid tumors including biliary tract, colorectal, pancreatic, esophageal, and breast cancers¹¹. The maximum tolerated dose was determined to be 60 mg/m²; AUC0-∞ increased between 12 and 25 mg/m², no difference was observed between 40 and 60 mg/m², and the recommended dose for successive Phase II trial was determined to be 25 mg/m². In a subgroup analysis of cancer types, the highest efficacy was achieved with BTC. In addition, Grade 3 hepatic dysfunction was observed only in two patients with the Rapid form of NAT2. NAT2 is the major liver metabolizing enzyme of Nanvuranlat, and the parent and metabolites are excreted in bile¹². The Rapid form, compared to the Non-Rapid form, has more metabolite (NAc-JPH203) than the Non-Rapid form.

### [Prior art references]

### [Patent documents]

Patent document 1: JPB 6734971
Patent document 2: JPB 6894155
Patent document 3: JPB 5826495

### [Summary of Invention]

### [Problems to be resolved by the invention]

The inventors of the present invention evaluated the efficacy and safety of LAT1 inhibitors, particularly Nanvuranlat, in patients with advanced biliary tract cancer who have a specific metabolic phenotype of NAT2, and failed or are refractory to standard chemotherapy, and found that it was highly effective against a specific subtype of biliary tract cancer.

Furthermore, the inventors evaluated the efficacy and safety of LAT1 inhibitors, particularly Nanvuranlat, in patients with curatively unresectable biliary tract cancer that has become refractory to cancer chemotherapy and has worsened and with high level LAT1 expression, especially with Non-Rapid NAT2, resulting in finding extremely excellent effects to achieve the present invention.

The present invention is to provide a biliary tract cancer treatment agent and biliary tract cancer treatment method. The present invention is also to provide a treatment agent and method effective for advanced biliary tract cancer that has a specific metabolic phenotype of NAT2 and is refractory or intolerant to standard chemotherapy, as well as certain subtypes of biliary tract cancer.

### [Means of solving the problems]

The present invention provides the following embodiments.

(Embodiment 1) A treatment agent for advanced biliary tract cancer that is refractory or intolerant of standard chemotherapy in a subject, comprising a LAT1 inhibitor.

(Embodiment 2) The treatment agent according to Embodiment 1, wherein the LAT1 inhibitor is O-(5-amino-2-phenylbenzoxazol-7-yl)methyl-3 ,5-dichloro-L-tyrosine, or a pharmaceutically acceptable salt thereof.

(Embodiment 3) The treatment agent according to Embodiment 1, wherein the advanced biliary tract cancer in the subject that is refractory or intolerant of standard chemotherapy is selected from the group consisting of extrahepatic biliary tract cancer and gall bladder cancer.

(Embodiment 4) The treatment agent according to Embodiment 3, wherein the biliary tract cancer is extrahepatic biliary tract cancer.

(Embodiment 5) The treatment agent according to Embodiment 3, wherein the biliary tract cancer is gall bladder cancer.

(Embodiment 6) The treatment agent according to Embodiment 3, wherein the subject is a patient with high LAT1 expression.

(Embodiment 7) The treatment agent according to Embodiment 3 or 6, wherein the subject is a subject identified as having a Non-Rapid (Slow and/or Intermediate) type NAT2 gene.

(Embodiment 8) The treatment agent according to Embodiment 1, wherein the subject is a patient with high LAT1 expression.

(Embodiment 9) The treatment agent according to Embodiment 8, wherein the biliary tract cancer is extrahepatic biliary tract cancer or gall bladder cancer.

(Embodiment 10) The treatment agent according to Embodiment 9, wherein the subject is a subject identified as having a Non-Rapid (Slow and/or Intermediate) type NAT2 gene.

(Embodiment 11) The treatment agent according to Embodiment 1, wherein the biliary tract cancer is intrahepatic biliary tract cancer.

(Embodiment 12) The treatment agent according to Embodiment 11, wherein the subject is a patient with high LAT1 expression.

(Embodiment 13) The treatment agent according to Embodiment 12, wherein the subject is a subject identified as having a Non-Rapid (Slow and/or Intermediate) type NAT2 gene.

(Embodiment 14) The treatment agent according to any one of Embodiments 1-3, 8 and 11, wherein 1-60 mg/m² of LAT1 inhibitor is administered to the subject.

(Embodiment 15) The treatment agent according to Embodiment 14, wherein 12.5 to 60 mg/m² of LAT1 inhibitor is administered to the subject.

(Embodiment 16) The treatment agent according to Embodiment 15, wherein 12.5 to 25 mg/m² of LAT1 inhibitor is administered to the subject.

(Embodiment 17) The treatment agent according to any one of Embodiments 1-3, 8 and 11, wherein a fixed amount of the LAT1 inhibitor to be administered to the subject is administered intravenously continuously over a fixed time period.

(Embodiment 18) The treatment agent according to Embodiment 17, wherein 100 mL of the LAT1 inhibitor is administered intravenously over 90 minutes.

(Embodiment 19) The treatment agent according to any one of Embodiments 1-3, 8 and 11, wherein the subject is a human.

(Embodiment 20) A pharmaceutical composition comprising a biliary tract cancer treatment agent according to Embodiment 1, and a pharmaceutically acceptable additive.

(Embodiment 21) A treatment agent for of curatively unresectable biliary tract cancer that has become refractory to cancer chemotherapy and has worsened in a subject, comprising a LAT1 inhibitor.

(Embodiment 22) The treatment agent according to Embodiment 21, wherein the LAT1 inhibitor is O-(5-amino-2-phenylbenzoxazol-7-yl)methyl-3,5-dichloro-L-tyrosine, or a pharmaceutically acceptable salt thereof.

(Embodiment 23) The treatment agent according to Embodiment 21, wherein the biliary tract cancer is selected from the group consisting of extrahepatic biliary tract cancer and gall bladder cancer.

(Embodiment 24) The treatment agent according to Embodiment 23, wherein the biliary tract cancer is extrahepatic biliary tract cancer.

(Embodiment 25) The treatment agent according to Embodiment 23, wherein the biliary tract cancer is gall bladder cancer.

(Embodiment 26) The treatment agent according to Embodiment 23, wherein the subject is a patient with high LAT1 expression.

(Embodiment 27) The treatment agent according to Embodiment 23 or 26, wherein the subject is a LAT1-high expressing patient with Non-Rapid N-acetyltransferase 2.

(Embodiment 28) The treatment agent according to Embodiment 21, wherein the subject is a patient with high LAT1 expression.

(Embodiment 29) The treatment agent according to Embodiment 28, wherein the biliary tract cancer is extrahepatic biliary tract cancer or gall bladder cancer.

(Embodiment 30) The treatment agent according to Embodiment 29, wherein the Non-Rapid type is Slow and/or Intermediate.

(Embodiment 31) The treatment agent according to Embodiment 21, wherein the biliary tract cancer is intrahepatic biliary tract cancer.

(Embodiment 32) The treatment agent according to Embodiment 31, wherein the subject is a patient with high LAT1 expression.

(Embodiment 33) The treatment agent according to Embodiment 32, wherein the subject is a LAT1-high expressing patient with Non-Rapid N-acetyltransferase 2.

(Embodiment 34) The treatment agent according to any one of Embodiments 21-23 and 28 and 31, wherein 1-60 mg/m² of LAT1 inhibitor is administered to the subject.

(Embodiment 35) The treatment agent according to Embodiment 34, wherein 12.5 to 60 mg/m² of LAT1 inhibitor is administered to the subject.

(Embodiment 36) The treatment agent according to Embodiment 35, wherein 12.5 to 25 mg/m² of LAT1 inhibitor is administered to the subject.

(Embodiment 37) The treatment agent according to any one of Embodiments 21-23 and 28 and 31, wherein a fixed amount of the LAT1 inhibitor to be administered to the subject is administered intravenously continuously over a fixed time period.

(Embodiment 38) The treatment agent according to Embodiment 37, wherein 100 mL of the LAT1 inhibitor is administered intravenously over 90 minutes.

(Embodiment 39) The treatment agent according to any one of Embodiments 21-23 and 28 and 31, wherein the subject is a human.

(Embodiment 40) A pharmaceutical composition comprising the treatment agent according to Embodiment 21, and a pharmaceutically acceptable additive.

Furthermore, the present invention provides the following embodiments.

(Embodiment 41) A treatment agent for curatively unresectable biliary tract cancer with high LAT1 expression with Non-Rapid N-acetyltransferase 2 that has become refractory to cancer chemotherapy and has worsened in the subject, including a LAT1 inhibitor.

(Embodiment 42) The treatment agent according to Embodiment 41, wherein the subject is a patient with LAT1-high expressing cancer.

(Embodiment 43) The treatment agent according to Embodiment 41 or Embodiment 42, wherein the biliary tract cancer is selected from the group consisting of extrahepatic biliary tract cancer, intrahepatic biliary tract cancer, and gall bladder cancer.

(Embodiment 44) A treatment method for an advanced biliary tract cancer that is refractory or intolerant of standard chemotherapy in a subject, comprising administering an agent containing an LAT1 inhibitor to the subject.

(Embodiment 45) Use of LAT1 inhibitors for the manufacture of pharmaceuticals for the treatment of advanced biliary tract cancer that is refractory or intolerant of standard chemotherapy in a subject.

(Embodiment 46) The treatment method of Embodiment 44 or use of Embodiment 45, wherein the LAT1 inhibitor is O-(5-amino-2-phenylbenzoxazol-7-yl)methyl -3,5-dichloro-L-tyrosine, or a pharmaceutically acceptable salt thereof.

(Embodiment 47) The treatment method of Embodiment 44 or use of Embodiment 45, wherein the subject is a patient with LAT1-high expressing cancer.

(Embodiment 48) The treatment method of Embodiment 44 or use of Embodiment 45, wherein the biliary tract cancer is selected from the group consisting of extrahepatic biliary tract cancer, intrahepatic biliary tract cancer and gall bladder cancer.

In one embodiment, the subject is a subject identified as having a Non-Rapid (Slow and/or Intermediate) type NAT2 gene.

Four subtypes of "advanced biliary tract cancer that is refractory or intolerant of standard chemotherapy" include IHC, EHC, GBC, and AVC. In the present invention, EHC and GBC among those four are highly preferred.

### [Effects of the Invention]

The present invention may provide a treatment method for biliary tract cancer with significantly improved efficacy response compared to other subtypes of biliary tract cancer, by administrating to a subject with advanced biliary tract cancer that has failed or is refractory to standard chemotherapy and has been identified as having a non-rapid (slow and/or intermediate) NAT2 gene, particularly extrahepatic bile duct cancer and gall bladder cancer.

### [Mode for carrying out the invention]

The following is a detailed description of the invention.

O-(5-amino-2-phenylbenzoxazol-7-yl)methyl-3,5 -dichloro-L-tyrosine (Nanvuranlat) The LAT1 inhibitor, an active ingredient of the present invention, may be preferably O-(5-amino-2-phenylbenzoxazol-7-yl) methyl-3,5-dichloro-L-tyrosine, named by the generic name Nanvuranlat, which is the compound disclosed in JPB 6734971 (Patent Document 1).

After intravenous administration, Nanvuranlat is metabolized (acetylated) mainly by hepatic NAT2 to an acetyl form (Nac-Nanvuranlat), which greatly reduces its bioactivity (Drug Metab Pharmacokinet. 2012; 27(1): 155 2012; 27(1):155-61).

In the present invention, Nanvuranlat can also be used as a pharmaceutically acceptable salt thereof. For example, the amino groups in the molecule of Nanvuranlat form salts together with acids. Such salts include, but are not limited, for example, salts with inorganic acids such as hydrochloric acid and sulfuric acid, and carboxylic acids. Among those, hydrochloric acid salts, especially mono hydrochloric acid salt monohydrate, are preferred.

### Biliary tract cancer treatment agent

The biliary tract cancer treatment agent of the present invention contains, as an active ingredient, an LAT1 inhibitor, preferably Nanvuranlat or a pharmaceutically acceptable salt thereof. If necessary, the biliary tract cancer treatment agent of the present invention can be made into a pharmaceutical composition by adding a pharmaceutically acceptable additive. Furthermore, the biliary tract cancer treatment agent or pharmaceutical composition can be administered orally in the form of solid preparations such as tablets, granules, fine granules, powders, capsules, or in the form of liquids, jellies, syrups, etc., or the treatment agent or pharmaceutical composition can be administered non-oral in the form of injectable, intranasal, suppository, inhalation, transdermal, etc.

The pharmaceutically acceptable additives of the present invention may include conventionally known additives such as excipients, binders, disintegrants, lubricants, surfactants, stabilizers, pH adjusters, emulsifiers, antioxidants, fillers, preservatives, sweeteners, flavor enhancers, dispersants, buffers, suspending agents, dissolution aids, and isotonic agents.

In particular, the biliary tract cancer treatment agent or pharmaceutical composition of the present invention may be preferably prepared as injectable formulations, since it is used as an anticancer agent. Examples of such injectable formulations in the present invention treatment agents include those containing pH adjusters and additives such as cyclodextrins.

Specifically, the injectable formulations may include, for example, intravenous, subcutaneous, intradermal, intramuscular, and intravenous injectable formulations.

Examples of pH adjusters that can be contained in the injectable formulations of the present invention include acids such as hydrochloric acid, citric acid, succinic acid, adipic acid, tartaric acid, lactic acid, fumaric acid, phosphoric acid; alkali metal hydroxides such as sodium carbonate, potassium carbonate, sodium hydroxide, potassium hydroxide; alkali metal hydrides such as sodium hydride and potassium hydride; and carbonates of alkali metals or alkaline earth metals, among those sodium hydroxide and sodium carbonate are preferred, and sodium hydroxide is more preferred.

The aforementioned injectable formulation can be suitably adjusted to a suitable pH using a pH adjuster. The pH of the injectable formulation in this embodiment may be preferably between 3 and 6, more preferably between 3 and 5, even more preferably between 3 and 4.5, and especially preferably between 3.5 and 4.5.

Examples of cyclodextrins that can be used in the present invention for injection include, for example, unmodified cyclodextrins and modified cyclodextrins. Examples of unmodified cyclodextrins include α-cyclodextrin, β-cyclodextrin, γ-cyclodextrin, and the like. Examples of modified cyclodextrins include dimethyl-α-cyclodextrin, dimethyl-β-cyclodextrin, dimethyl-γ-cyclodextrin, and hydrophilic cyclodextrin. -cyclodextrin, hydroxypropyl-α-cyclodextrin, hydroxypropyl-β-cyclodextrin, hydroxypropyl γ-cyclodextrin, sulfobutyl ether-α-cyclodextrin, sulfobutyl ether-β-cyclodextrin, sulfobutyl ether-β-cyclodextrin sulfobutyl ether-γ-cyclodextrin, maltosyl-α-cyclodextrin, and the like.

Cyclodextrins may be used alone or in any combination of two or more. From the viewpoints that cyclodextrins reduce the number of insoluble particles formed even when dissolved in non-acidic aqueous solutions and improve the redissolubility of the lyophilized formulation in non-acidic aqueous solutions, hydroxypropyl-β cyclodextrin and sulfobutyl ether-β-cyclodextrin are preferred, and sulfobutyl ether-β-cyclodextrin is more preferred.

Here, sulfobutyl ether cyclodextrin has the structure shown in Formula 1 below, and the inside of the ring structure is highly hydrophobic. Therefore, O-(5-amino-2-phenylbenzoxazol-7-yl)methyl 3,5-dichloro-L-tyrosine, which is also highly hydrophobic, form a complex with 3,5-dichloro-L-tyrosine through hydrophobic interactions. References in the specification to "sulfobutyl ether cyclodextrin (SBECD) complexes" refer to those due to the hydrophobic interactions described above.

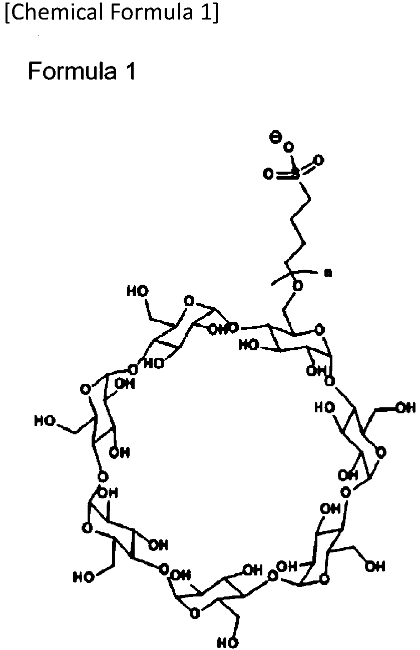

Alternatively, the active ingredient, Nanvuranlat, may be used in the injectable formulation as its sulfobutyl ether cyclodextrin complex ("Nanvuranlat-SBECD").

The injectable formulation of the present invention may further include, as needed, buffers, suspending agents, dissolution aids, stabilizers, isotonic agents, preservatives, and the like.

Examples of buffers include boric acid buffers, phosphate buffers, citrate buffers, acetate buffers, and Tris buffers.

Examples of suspending agents include methylcellulose, polysorbate 80, hydroxyethylcellulose, gum arabic, tragacanth powder, sodium carboxymethylcellulose, polyoxyethylene sorbitan monolaurate, poloxamer, hydroxypropyl methylcellulose (HPMC), and sodium alginate.

Examples of dissolution aids include polyoxyethylene hardened castor oil, polysorbate 80, nicotinic acid amide, polyoxyethylene sorbitan monolaurate, macrogol, glycerol fatty acid esters, lipoaminoacid, and polyethylene glycol.

Examples of stabilizing agents include sodium sulfite, sodium metabisulfite, and the like; examples of isotonic agents include glycerin, sodium chloride, and the like; and examples of preservatives include methyl paraoxibenzoate, ethyl paraoxibenzoate, sorbic acid, and the like.

The pH of the lyophilized formulation when dissolved in water may be preferably between 3 and 6, more preferably between 3 and 5, even more preferably between 3 and 4.5, and especially preferably between 3.5 and 4.5.

The lyophilized formulation may be produced by a method for producing known lyophilized formulations, for example, the method includes freezing at a temperature of -25°C or less, followed by maintaining a vacuum of approximately 20 Pa or less and drying while raising the temperature up to room.

The injectable formulation of the present invention may be a lyophilized formulation. Such lyophilized formulations can be used by dissolving at the time of use in one or more of the solvents from the group consisting of distilled water for injection, infusion solution, and electrolyte solution.

The treatment agent and method of treatment of the present invention are effective for advanced biliary tract cancer that is refractory or intolerant of standard chemotherapy, and particularly highly effective for IHC, EHC, and GBC among the four subtypes of BTC such as IHC, EHC, GBC, and AVC.

### Medication Regimen

The dosing regimen to which the biliary tract cancer treatment agent of the present invention may be applied is selected according to various factors, including patient type, race, age, weight, sex, and medical condition; severity of the condition to be treated; route of administration; and liver and kidney function of the patient. The physician can easily determine and prescribe the effective dose of the drug needed to prevent, block or stop the progression of the condition.

As used herein, the terms "subject" and "patient" are used interchangeably and are preferably for humans. In particular, a "patient" is preferably a patient with a Non-Rapid (Slow and/or Intermediate) genetic type of NAT2, as described below.

In one embodiment, a patient is a biliary tract cancer patient who is refractory or intolerant of standard chemotherapy and trial drugs (pre-treatment), or has advanced biliary tract cancer that is refractory or intolerant of standard chemotherapy.

In the biliary tract cancer treatment agent and treatment method of the present invention, a dosage of the active ingredient (LAT1 inhibitor) can be selected according to the degree of symptoms, patient age, sex, weight, sensitivity difference, timing of administration, administration interval, and the like. Examples of dosage from the perspective of efficacy and safety include 1 mg/m² to 60 mg/m² (body surface area) per time, preferably 12.5 mg/m² to 60 mg/m², 12.5 mg/m² to 25 mg/m², or 10 mg/m² to 40 mg/m² (body surface area), and more preferably 25 mg/m². The dose may be reduced to 12.5 mg/m² depending on symptoms.

Examples of the administration regimen of the biliary tract cancer treatment agent and treatment method of the present invention include;
- Biliary tract cancer treatment agent is administered in a cycle of continuous administration for a fixed period of time followed by a fixed period of rest,
- Biliary tract cancer treatment agent is administered for one cycle of 14 days, consisting of 5 consecutive days of administration followed by a 9-day rest period,
- A certain amount of biliary tract cancer treatment agent is administered intravenously continuously over a certain period of time,
- 100 mL of biliary tract cancer treatment agent is administered continuously intravenously over 90 minutes,
- Biliary tract cancer treatment agent of 1-60 mg/m² per patient is administered,
- Biliary tract cancer treatment agent of 12.5 to 60 mg/m² per patient is administered,
- Biliary tract cancer treatment agent of 12.5-25 mg/m² per patient is administered, and
- Biliary tract cancer treatment agent of 25 mg/m² per patient is administered, or a combination of the above can be applied as needed.

The biliary tract cancer treatment agent and treatment method of the present invention can be highly effective when applied to patients with Non-Rapid (Slow and/or Intermediate) type NAT2 gene (i.e., slow type of acetylation by NAT2). As used herein, the term "Non-Rapid NAT2 gene" refers to the intermediate and slow phenotypes of the three phenotypes of the NAT2 gene, namely, Rapid, Intermediate, and Slow acetylation rate.

NAT2 acetyl phenotypes of seven SNPs (481C>T, 282C>T, 857G>A, 803A>G, 341T>C, 590G>A, 191G>A) representing the genetic polymorphism of NAT2 were analyzed by the combination of 2-SNP, 3-SNP, and 4-SNP, the one consisting of homozygotes of NAT2*4 without SNP (standard) haplotype was considered as Rapid acetylation type (Rapid), the heterozygote with one of the haplotypes mutated was considered as Intermediate acetylation type (Intermediate), and the homozygous including only mutant haplotypes or a combination of heterozygous including two or more mutant haplotypes was considered the slow acetylation type (Slow) (Pharmacogenomics, vol. 13, pp. 31 -41 pages, 2012). Here, the "NAT2*4" allele refers to the SNP-free haplotype. According to Pharmacogenomics, vol. 13: pp. 31-41, 2012, in addition to "NAT2*4", Rapid type also includes patients with "NAT2*11, NAT2*12, and NAT2*13" without amino acid mutations.

Specifically, the NAT2 acetylation factor phenotype inferred by two SNPs is determined by analysis of two SNPs: rs1041983 (282C>T) and rs1801280 (341T>C). The NAT2 acetylation factor phenotype inferred by three SNPs: rs 1799929 (481C>T), rs1799930 (590G>A) and rs1799931 (857G>A). 4 SNP inferred NAT2 acetylation factor phenotype is determined by analysis of 4 SNPs: rs1801279 (191G>A), rs1801280 (341T> C), rs1799930 (590G>A), and rs1799931 (857G>A).

When Nanvuranlat is acetylated by NAT2, its inhibitory activity against cancer cells is reduced. However, if the patient has a predominant NAT2 gene phenotype that is less susceptible to acetylation, namely, if the NAT2 gene of the patient, such as cancer, has an Intermediate or Slow acetylation phenotype (Non-Rapid type NAT2 gene), the acetylation rate of Nanvuranlat is slower, resulting in less acetylation and longer-lasting Nanvuranlat activity (JPB 6894155).

The biliary tract cancer treatment agents and method of treating biliary tract cancer may respectively be combined with other cancer therapies. Such cancer therapies include, for example, chemotherapy, targeted therapies such as antibody therapy, immunotherapy, and radiation therapy.

### [Examples]

Hereinafter, the present invention is described with reference to specific embodiments, but the present invention is not limited to the embodiments, and it is understood that various changes and modifications thereof may be made by those skilled in the art without departing from the scope or intent of the invention as set forth in the appended claims.

### Example 1

As Phase II trial of Nanvuranlat, a multicenter, randomized, placebo-controlled, double-blind, parallel-group Phase II trial was conducted at 14 centers in Japan for patients with advanced biliary tract cancer who were refractory or intolerant of standard treatment. Patients were randomized in a 2:1 ratio to receive the drug or placebo using biliary tract cancer (BTC) subtype and resection status as allocation factors. The non-rapid (Slow and/or Intermediate) genotype was set as the NAT2 genotype for selection criteria, and based on the results of the PI study conducted earlier, 25 mg/m2 of Nanvuranlat or placebo was administered intravenously for 5 consecutive days and rested for 9 days (14 days: 1 cycle). Administration of the trial drug was continued until the discontinuation criteria were met. The primary endpoint was progression-free survival using RECIST 1.1 based on Blinded Independent Central Review. Images were obtained every 6 weeks from the start date and at discontinuation. PFS was defined as the date of the first documented progressive disease (PD) or death for any reason after randomization. The primary evaluation was conducted in the full analysis set (FAS) population, which consisted of all biliary tract cancer patients randomized to one of the arms. The safety evaluation was conducted in the SAFETY population, which was randomly assigned to one of the arms and received the trial drug. Nanvuranlat was used as monohydrochloride monohydrate.

### Efficacy Endpoints

A total of 106 biliary tract cancer patients (ITT (intention to treat) population) were randomized to receive either the Nanvuranlat (n=70) or placebo (n=36). The FAS population included 104 patients (Nanvuranlat group: n=69, placebo group: n=35). The breakdown of NAT2 metabolic phenotypes in the FAS population was NAT2 Non-Rapid (81 patients), NAT2 rapid (5 patients), and NAT2 unknown (20 patients).

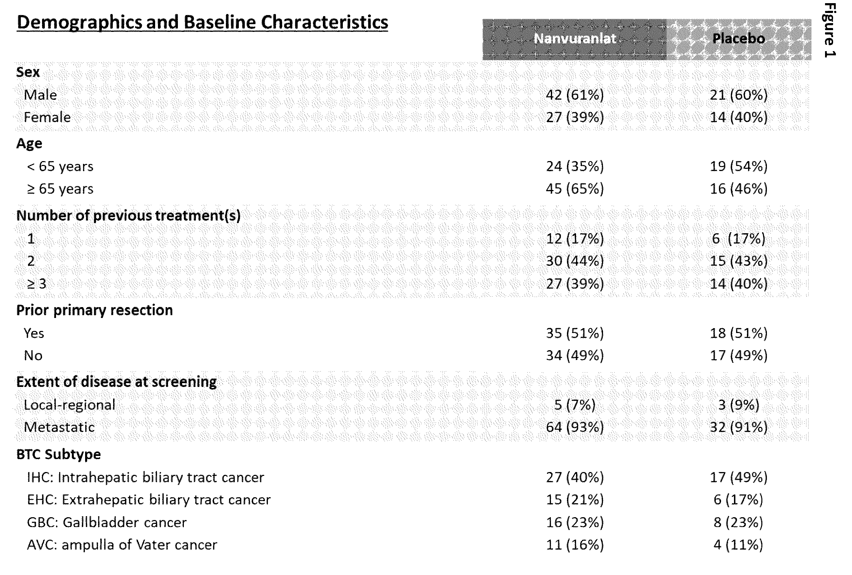

The background of the biliary tract cancer patients assigned to the FAS population in this trial is shown in Figure 1. Biliary tract cancer is an anatomically diverse disease, but in this trial, all subtypes of biliary tract cancer (intrahepatic biliary tract cancer: IHC (intrahepatic), extrahepatic biliary tract cancer: EHC (extrahepatic), gallbladder cancer: GBC (gall bladder), and ampulla of Vater (AVC)) were included. The characteristics of the enrolled patients were as follows: 1) 83% of the patients were enrolled in the trial as third-line treatment or later, and the majority of the patients had terminal cancer for which palliative treatment was considered, and 2) approximately half of the patients were aged 65 years or older. More than 98% of the patients enrolled in the trial had distant metastasis, and the majority of patients were under salvage therapy with no effective treatment options.

Biliary tract cancer is an anatomically diverse disease, but in this trial, all subtypes of biliary tract cancer (intrahepatic biliary tract cancer: IHC (intrahepatic), extrahepatic biliary tract cancer: EHC (extrahepatic), gallbladder cancer: GBC (gall bladder), and ampulla of Vater (AVC)) were included.

Biliary tract cancer is an anatomically diverse disease, but in this trial, all subtypes of biliary tract cancer (intrahepatic biliary tract cancer: IHC (intrahepatic), extrahepatic biliary tract cancer: EHC (extrahepatic), gallbladder cancer: GBC (gall bladder), and ampulla of Vater (AVC)) were included.

### Evaluation Method:

### (1) Estimation of hazard ratio (HR) by stratified Cox proportional hazards model

Kaplan-Meier estimates corresponding to the event time points are calculated and plotted for each treatment group. Confidence intervals for the Kaplan-Meier estimates are constructed using the log-log method with standard errors approximated by the greenwood formula. Point estimates at the 25%, 50%, and 75% of survival time and 95% confidence intervals are obtained and presented in tabular form. The Brookmeyer and Crowley method is used to construct the confidence intervals for survival time percentiles.

### Stratified log-rank Test

The p-value of the score test in the above model fit is calculated and presented along with the corresponding χΛ2 statistic and degrees of freedom.

### (2) Estimation of hazard ratios using a stratified Cox proportional hazards model with the addition of an interaction term for NAT2 metabolic type

Fitting to a stratified Cox proportional hazards model, the hazard ratio exp((β_drug)Λ) and its 95% confidence interval are estimated and presented in tabular and forest plot form, along with the p-value of the Wald test corresponding to the null hypothesis "β_drug=0; exp(β_drug)=1".
Stratification factor 1: "Facility," "Gall bladder cancer and other cancers," and "Existence or nonexistence of primary tumor resection history"
Stratification factor 2: "Biliary tract cancer subtype (intrahepatic biliary tract cancer, extrahepatic biliary tract cancer, biliary tract cancer, or duodenal papillary carcinoma)" and " Existence or nonexistence of primary tumor resection history"

### Test Results

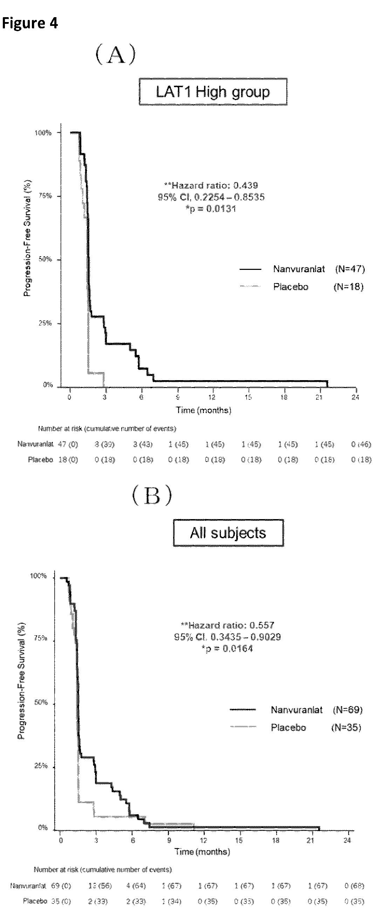

The Nanvuranlat showed a statistically significant benefit over placebo in the primary endpoint of PFS by blinded, independent central imaging evaluation [hazard ratio (HR) = 0.557; 95%Cl: 0.3435-0.9029; p value = 0.0164]. Since 83% of the subjects enrolled in the trial were in the terminal stage, 70% of those treated with Nanvuranlat and placebo experienced a PFS event within approximately one month after the start of treatment, resulting in a sharp decline in progression-free survival, with a Median PFS of 46.0 days and 43.1 days for Nanvuranlat and placebo groups, respectively, and no difference in Median PFS between the two groups. Approximately 70% of the time had passed since the start of administration when the group that responded to Nanvuranlat began to appear, the progression-free survival rate at 1.5 months after the start of treatment was 53.6% (37/69 cases) in the Nanvuranlat group compared to 40.0% (14/35 cases) in the placebo group, and the rate at 3 months, respectively, was 14.5% (10/69 cases) compared to 5.7% (2/35 cases) (Figure 4 (B)).

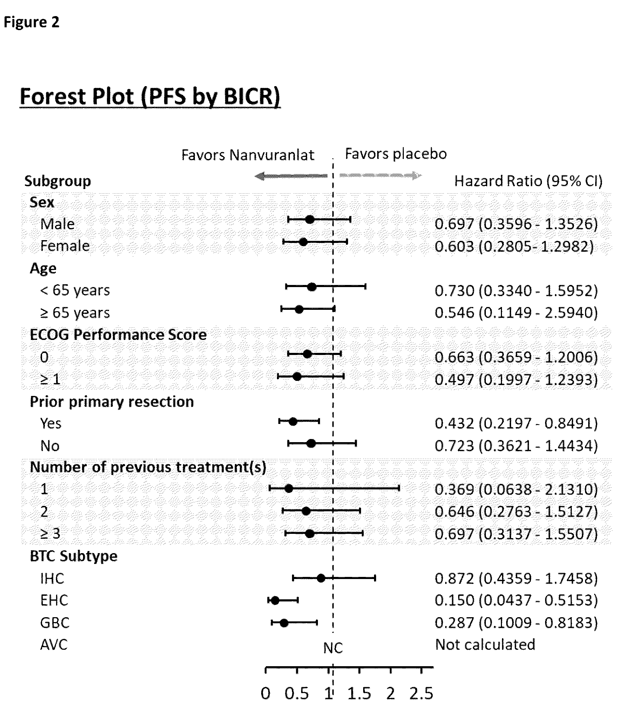

Subpopulation analysis using imaging data consistently showed favorable hazard ratios for the Nanvuranlat group versus the placebo group in almost all predefined subgroups. In particular, the results suggested a benefit of Nanvuranlat in EHC and GBC among biliary tract cancer subtypes, and in cases of primary resection (Figure 2). The HR values were 0.872 (0.4359-1.7458) for IHC, 0.150 (0.0437-0.5153) for EHC, 0.287 (0.1009-0.8183) for GBC, and AVC: not computable, and it was found that EHC and GBC were significantly more effective than the other subtypes.

### Safety Endpoints

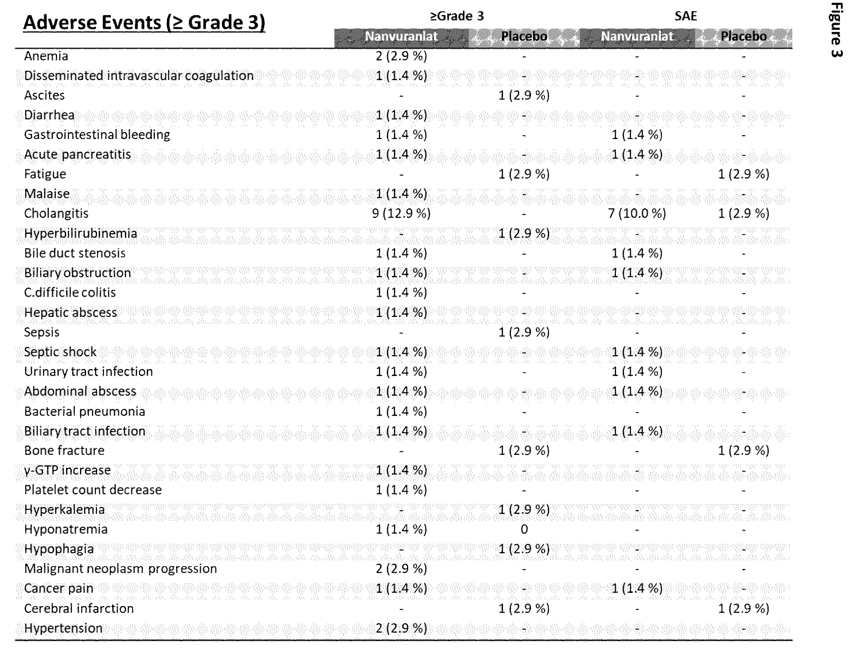

There were no deaths suspected to be related to Nanvuranlat throughout the trial observation period. Also, there were no discontinuations or dose reductions of Nanvuranlat. The frequency of all adverse events that occurred during the trial period was 85.7% (60/70) and 82.9% (29/35) in the Nanvuranlat and placebo groups, respectively. All adverse events of Grade 3 or higher occurred in the Nanvuranlat group (30.0%) versus the placebo group (22.9%). Adverse events undeniably related to Nanvuranlat were 41.4% in the Nanvuranlat group and 57.1% in the placebo group. All adverse events of Grade 3 or higher and SAEs are shown in Figure 3.

Among these adverse events, there were three undeniably related Grade 3 or higher adverse events including one case of fatigue, one case of decreased platelet count, and one case of hypertension. In the placebo group, there were three cases including one case of ascites, one case of fatigue, and one case of decreased appetite. One serious adverse event was fatigue in the placebo group.

The fact that the efficacy of the drug was extremely high in extrahepatic biliary tract cancer (EHC) and gallbladder cancer (GBC), which are generally considered to have a poor prognosis, is an unexpected result. In particular, the hazard ratio for extrahepatic biliary tract cancer is approximately twice that for gall bladder cancer, indicating that the drug is extremely effective.

### Example 2

The following randomized Phase II trial is investigating the efficacy and safety of Nanvuranlat in patients with advanced BTC who have already received one or more systemic chemotherapy regimens.

### Test design

This test was a multicenter, randomized, double-blind, placebo-controlled, Phase II trial conducted at 14 major cancer centers/hospitals in Japan. Patients were randomly assigned to the Nanvuranlat or placebo group (2:1), stratified by BTC subtype and whether the primary cancer lesion had been resected, generated by an independent statistical group. Randomization to the two treatment groups was performed by IWRS (Interactive Web-based Response System) and generated by an independent statistical group.

Nanvuranlat 25 mg/m²/day or placebo was administered intravenously for 5 consecutive days, followed by a 9-day rest period (=14 days: 1 cycle). The trial drug was administered by 90-minute infusion at the investigator's site (all patients were admitted during the first treatment cycle). Patients, drug administrators, and clinical trial coordinators were blinded to trial treatment. Nanvuranlat was used as monohydrochloride monohydrate.

Treatment with additional cycles was to continue until disease progression, the occurrence of other unacceptable toxicities, pregnancy confirmation, death, withdrawal of consent, loss to follow-up, unblinding of the trial, or termination of the trial, as determined by the investigator. Data cutoff was defined as the date of completion of 6 months of treatment of the last patient entry into the trial. Patient survival was followed until the 95th percentile event or 9 months after the data cutoff, whichever occurred first.

### Clinical Trial Monitoring

The trial was conducted in accordance with the principles of the International Conference on Harmonization of Good Clinical Practice Guidelines and the Declaration of Helsinki. Approval from the Clinical Trials Review Committee and/or the Clinical Trials Ethics Committee was obtained at each trial site. Patients provided written informed consent before participating in the clinical trial. The clinical trial was designed by the sponsor with input from the Steering Committee and established through communication with the Pharmaceuticals and Medical Devices Agency (PMDA) at the Regulatory Science Strategy Meeting.

### Patients

Appropriate patients are 20 years of age or older with advanced BTC confirmed by image contrast or surgical findings in the past medical history and refractory or intolerant to standard chemotherapy and/or trial drugs. In the case of EHC, GBC, and AVC, adrenal carcinoma or adenosquamous carcinoma was histologically confirmed; in the case of IHC, adrenal carcinoma was histologically confirmed. Patients with unresectable or recurrent BTC were appropriate for this trial and provided FFPE (formalin-fixed, paraffin-embedded) tumor specimens. Additional primary eligibility criteria included a life expectancy of at least 90 days;
Eastern Cooperative Oncology Group (ECOG) performance status score of 0 or 1;
Measurable lesions (imaged within 4 weeks prior to enrollment) as defined by the Response Evaluation Criteria for Solid Tumors (RECIST) version 1.1;
Adequate hematological, liver and kidney function.

Based on the clinical findings of the Phase I exploratory analysis that the metabolism of Nanvuranlat by NAT2 affects the clinical outcome of patients¹¹, this Phase II trial protocol was modified after the first 25 patients were enrolled. Patients with Non-Rapid (Slow and Intermediate) forms of NAT2 were then enrolled by pre-defined NAT2 genotype assays according to a validated SNP assay procedure¹³. Personal information was kept strictly confidential at the trial site.

Patients with comorbidities such as cardiac disease including cerebrovascular disease, respiratory infarction, myocardial infarction, unstable angina, myocardial infarction, psychiatric disease, and drug or alcohol dependence were excluded. Additional exclusion criteria included active infection requiring systemic therapy, HCV-RNA positive hepatitis (known HIV antibodies, HB antigen), clinically abnormal electrocardiogram, ascites/pleural/pericardial effusion requiring drainage therapy, brain metastases, history of radiation to hematopoietic bone, colitis disease, shock or anaphylaxis to sulfobutyl ether-beta-cyclodextrin.

### Endpoints

The primary endpoint was progression-free survival (PFS) as assessed by blinded independent central review (BICR) using RECIST version 1.1. PFS was defined as the time from the date of randomization to the first recorded date of disease progression or death from any cause, whichever occurred first. The PFS was defined as the time from the date of randomization to the date of first documented disease progression or death from any cause, whichever occurred first. Radiographic evaluation (CT) for assessment of disease response was performed every 6 weeks (at the end of every 3 cycles of treatment or at the end of the trial plus or minus 3 days) according to RECIST 1.1 throughout the trial period. BICR did not confirm radiographic progression, which was determined locally, in real time.

Secondary endpoint was overall survival (OS), disease control rate (DCR) as assessed by BICR, and safety. Adverse events were graded using MedDRA/J version 24.1. Blood and urinary concentrations of Nanvuranlat and its metabolite N-acetyl-JPH203 (NAc-JPH203) were evaluated.

Tissue expression levels of LAT1 were determined by immunohistochemical methods and evaluation was performed according to established methods⁷.

Specifically, the mild variant of Sinicrope's method 3 was employed to evaluate the immunoreactivity of LAT1. That is, based on the immunoreactivity of cancer cell membranes, four categories were defined as follows. Expression intensity 0, no reaction; 1, weakly or spot positive; 2, moderately positive throughout the plasma membrane; 3, strongly positive throughout the plasma membrane. The highest intensity of LAT1 expression in the cancer tissue was used in each case. In addition, the region of LAT1 expression was expressed as a percentage of the total cancer tissue observed. The LAT1 expression score was expressed as the numerical value of LAT1 expression intensity X expression region (Patent document 3).

Therefore, the outline of the immunodepletion LAT1 determination method in biliary tract cancer is as follows.

**[Table 1]**

| | **Phase II** | | |
|---|---|---|---|
| Positive identification method | Strength | Positive rate (Area) | Score |
| | 1, Weak | 1, 1 to 10% | Strength x Positive Rate (Area) |
| | 2, Moderate | 2, 11 to 29% | |
| | 3, Strong | 3, ≧30% | |
| Definition of high expression | Score 4 or higher | | |
| Definition of low expression | Score 1-3 | | |

### Statistical analysis

Assuming a hazard ratio (HR) of 0.5 for PFS, more than 81 progression-free survival events would be needed to provide β: 90% power with a one-sided alpha level significance of 0.025 to reject the null hypothesis.

The primary efficacy analysis was performed using a full analysis set (FAS) population that included all randomly assigned BTC patients within their designated treatment group. The safety analysis population included all patients who received at least one trial treatment (safety population).

Cox hazard models were used to estimate HRs and 95% confidence intervals (Cls) for PFS and OS comparisons between the Nanvuranlat and placebo groups. All time-to-event endpoints were estimated by the Kaplan-Meier method. A stratified log-rank test was used to assess statistical significance. Subgroup analysis by previous line of therapy, gender, metastatic site at screening, BTC subtype, and ECOG performance status score was performed for PFS according to BICR.

Statistics were used to summarize safety data, response rates, pharmacokinetic and pharmacodynamic data. Unless otherwise specified, all reported p-values are one-sided. Statistical analysis was performed using SAS software (version 9.4).

### Result

### Patients

A total of 211 patients were enrolled in the trial, including 106 patients (82 Non-Rapid form of NAT2, 4 Rapid form of NAT2, and 20 unknown NAT2). 106 patients were randomized to the Nanvuranlat (n = 70) or placebo (n = 36) group. Primary efficacy analysis was performed in the FAS population (total n = 104: n = 69 for Nanvuranlat and n = 35 for placebo), where one patient assigned to Nanvuranlat was excluded due to the discovery of non-biliary tract cancer and one patient assigned to placebo dropped out before starting treatment.

Patient background and baseline characteristics were similar in the Nanvuranlat and placebo groups, except that Nanvuranlat patients were slightly older than the placebo (65% of patients 65 years and older for Nanvuranlat and 46% for placebo) and the number of IHC patients was slightly higher for placebo (Table 2). Most patients had received at least a second line of therapy prior to enrollment (83% in both groups).

**[Table 2]**

| Table 2. Patient background and baseline characteristics | | | | | |
|---|---|---|---|---|---|
| **Group** | | **Nanvuranlat** | | **Placebo** | |
| Number of subjects | | **69** | | **35** | |
| **Gender** | | | | | |
| | Male | 42 | 60.9% | 21 | 60.0% |
| | Female | 27 | 39.1% | 14 | 40.0% |

| **Age** | | | | | |
|---|---|---|---|---|---|
| | <65 years old | 24 | 34.8% | 19 | 54.3% |
| | ≧65 years old | 45 | 65.2% | 16 | 45.7% |

| **Performance Status Scores** | | | | | |
|---|---|---|---|---|---|
| | 0 | 45 | 65.2% | 24 | 68.6% |
| | 1 or more | 24 | 34.8% | 11 | 31.4% |

| **Previous primary resection** | | | | | |
|---|---|---|---|---|---|
| | Having | 36 | 52.2% | 18 | 51.4% |
| | Nothing | 33 | 47.8% | 17 | 48.6% |

| **Number of previous treatments** | | | | | |
|---|---|---|---|---|---|
| | 1 | 12 | 17.4% | 6 | 17.1% |
| | 2 | 30 | 43.5% | 15 | 42.9% |
| | 3 or more | 27 | 39.1% | 14 | 40.0% |

| **Extent of disease at screening** | | | | | |
|---|---|---|---|---|---|
| | Local-area | 5 | 7.2% | 3 | 8.6% |
| | Displacement | 64 | 92.8% | 32 | 91.4% |

| **BTC Subtype** | | | | | |
|---|---|---|---|---|---|
| Intrahepatic biliary tract cancer (IHC) | | 27 | 39.1% | 17 | 48.6% |
| Extrahepatic biliary tract cancer (EHC) | | 15 | 21.7% | 6 | 17.1% |
| Gall bladder cancer (GBC) | | 16 | 23.2% | 8 | 22.9% |
| Ampulla of Vater cancer (AVC) | | 11 | 15.9% | 4 | 11.4% |

### Efficacy analysis between LAT1 high expression subgroup and all patients

### Immunohistochemical staining

For patients with archival tissue available, LAT1 expression was evaluated in tumor biopsy samples. Formalin-fixed paraffin-embedded tissue was sliced into thin sections (3-4 micrometers thick) and deparaffinized samples were incubated with anti-LAT1 antibody overnight at 4°C. LAT1 expression was assessed microscopically by a defined score, which is a multiplication of the positivity and the number of cells distributed in the microscopic field of view.

LAT1 high-expressing subgroup vs. background of all patients

There are no significant differences in patient background between the LAT1 high-expressing subgroup and all LAT1 patients (Table 3).

**[Table 3]**

| | | **LAT1 high expression subgroup N = 65** | | | | **All LAT1 patients N = 104** | | | |
|---|---|---|---|---|---|---|---|---|---|
| **Group** | | **Nanvuranlat** | | **Placebo** | | **Nanvuranlat** | | **Placebo** | |
| Number of subjects | | **47** | | **18** | | **69** | | **35** | |
| **Gender** | | | | | | | | | |
| | Male | 28 | 59.6% | 11 | 61.1% | 42 | 60.9% | 21 | 60.0% |
| | Female | 19 | 40.4% | 7 | 38.9% | 27 | 39.1% | 14 | 40.0% |

| **Age** | | | | | | | | | |
|---|---|---|---|---|---|---|---|---|---|
| | < 65 years old | 15 | 31.9% | 10 | 55.6% | 24 | 34.8% | 19 | 54.3% |
| | ≧65 years old | 32 | 68.1% | 8 | 44.4% | 45 | 65.2% | 16 | 45.7% |

| **Performance Status Scores** | | | | | | | | | |
|---|---|---|---|---|---|---|---|---|---|
| | 0 | 29 | 61.7 | 8 | 44.4 | 45 | 65.2% | 24 | 68.6% |
| | 1 or more | 18 | 38.3 | 10 | 55.6 | 24 | 34.8% | 11 | 31.4% |

| **Previous primary resection** | | | | | | | | | |
|---|---|---|---|---|---|---|---|---|---|
| | Having | 24 | 51.1% | 10 | 55.6% | 35 | 50.7% | 18 | 51.4% |
| | Nothing | 23 | 48.9% | 8 | 44.4% | 34 | 49.3% | 17 | 48.6% |

| **Number of previous treatments** | | | | | | | | | |
|---|---|---|---|---|---|---|---|---|---|
| | 1 | 8 | 17.0% | 2 | 11.1% | 12 | 17.4% | 6 | 17.1% |
| | 2 | 20 | 42.6% | 9 | 50.0% | 30 | 43.5% | 15 | 42.9% |
| | 3 or more | 19 | 40.4% | 7 | 38.9% | 27 | 39.1% | 14 | 40.0% |

| **Extent of disease at screening** | | | | | | | | | |
|---|---|---|---|---|---|---|---|---|---|
| | Local-area | 2 | 4.3 | 1 | 5.6 | 5 | 7.2% | 3 | 8.6% |
| | Displacement | 45 | 95.7 | 17 | 94.4 | 64 | 92.8% | 32 | 91.4% |

| **BTC Subtype** | | | | | | | | | |
|---|---|---|---|---|---|---|---|---|---|
| | Intrahepatic biliary tract cancer (IHC) | 17 | 36.2% | 6 | 33.3% | 27 | 39.1% | 17 | 48.6% |
| | Extrahepatic biliary tract cancer (EHC) | 12 | 25.5% | 4 | 22.2% | 15 | 21.7% | 6 | 17.1% |
| | Gall bladder cancer (GBC) | 12 | 25.5% | 5 | 27.8% | 16 | 23.2% | 8 | 22.9% |
| | Ampulla of Vater cancer(AVC) | 6 | 12.8% | 3 | 16.7% | 11 | 15.9% | 4 | 11.4% |

### Efficacy Results

### Primary Endpoint: PFS by BICR

A significant difference was observed between the Nanvuranlat and placebo groups (hazard ratio = 0.557; 95%CI: 0.3435-0.9029, p value: 0.0164 (one-sided) (Figure 4(B)). The PFS at 1.5 and 3 months were 43/69 (62.3%) and 16 (23.2%) for Nanvuranlat, compared with 14/35 (40.0%) and 2 (5.5%) for placebo. The median PFS was 46.0 days for Nanvuranlat and 43.0 days for the placebo group.

The level of LAT1 expression in tumor tissue was identified as a determinant of response to Nanvuranlat. Ninety eight samples (94.2%) from 104 patients were available for immunohistochemical LAT1 analysis. Of those, 65 patients (62.5%) were identified as high LAT1 expression (score: 4-9) (Table 4). Kaplan-Meier analysis of the LAT1 high expression subgroup compared to the analysis of all patients (HR=0.557, p=0.0164), showed a further improvement in the hazard ratio in the Nanvuranlat group (HR=0.439; 95%CI: 0.2254-0.8535; p=0.0131, Figure 4(A)).

**[Table 4]**

| Table 4. Distribution of high and low expression for LAT1 expression | | | | | |
|---|---|---|---|---|---|
| Entirety (FAS) | | Nanvuranlat | | Placebo | |
| Entirety | Scores | n = 69 | % | n = 35 | % |
| High expression | 4 to 9 | n = 47 | 68.1 | n = 18 | 51.4 |
| Low expression | 0 to 3 | n = 17 | 24.6 | n = 16 | 45.7 |
| Unidentified | | 5 | 7.2 | 1 | 2.9 |

Figure 4 shows the primary PFS by BICR in all patient groups of FAS (n=104: Figure 4(B)) (including 69 and 35 in the Nanvuranlat and placebo, respectively), and in the LAT1 high-expressing group (n=65, Figure 4(A)). Forty-seven and 18 patients are included in the Nanvuranlat and placebo groups, respectively. Kaplan-Meier plots of the probability of progression-free survival for the Nanvuranlat patients were compared to placebo patients. Hazard ratios were calculated by a stratified Cox proportional hazards model, and P values were calculated by a stratified log-rank test.

### Subgroup analysis by PFS with BICR

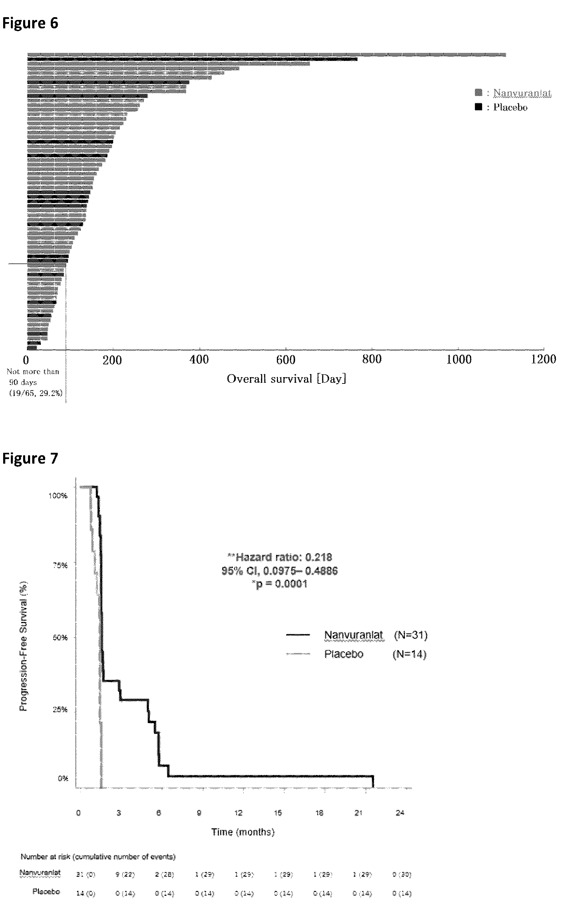

Nanvuranlat showed consistent efficacy across predefined subgroups (Figure 2). Efficacy was particularly high in patients with EHC and GBC. EHC and GBC have the same origin², so they were integrated in the subgroup analysis of PFS. PFS was significantly better for patients treated with Nanvuranlat versus placebo (HR: 0.218, Cl95%: 0.0975 -0.4886, p=0.0001; Figure 7).

### Secondary endpoint

### Overall survival rate

Although not statistically significant, in a full population analysis of FAS, the hazard ratio favored Nanvuranlat (HR: 0.849, 95%Cl: 0.5308-1.3574, p=0.4934) (Figure 4(A)). When only patients with high LAT1 were analyzed, OS -HR was in favor of Nanvuranlat (HR: 0.670, Cl95%: 0.3463-1.2954, p=0.2313) (Figure 4(B)).

The disease control rate (DCR: CR + PR + SD) was 24.6% in the Nanvuranlat group and 11.4% in the placebo group, and of the 17 disease control patients in the Nanvuranlat group, 11 patients (64.7%) were associated with high LAT1, while of the 4 disease control patients in the placebo group, only one patient had high LAT1 (25.0%) (Table 5). The difference was primarily driven by SD. In the Nanvuranlat group, DCRs were similar across BTC subtypes, including IHC, EHC, GBC, and AVC. DCRs were zero in the placebo group, except for the IHC subtype. The four IHC patients whose disease was controlled in the placebo group were those whose primary lesions had been resected.

**[Table 5]**

| Table 5. Distribution of DCR patients with high LAT1 and low LAT1 expression | | | | | |
|---|---|---|---|---|---|
| LAT1 expression | | Number of patients with controlled diseases (n = 21) | | | |
| | | Nanvuranlat | | Placebo | |
| LAT1 | Scores | 17 | % | 4 | % |
| High expression | 9 | 5 | 64.7 | 1 | 25.0 |
| | 6 | 4 | | 0 | |
| | 4 | 2 | | 0 | |
| Low expression | 3 | 0 | 17.6 | 0 | 75.0 |
| | 2 | 0 | | 1 | |
| | 1 | 2 | | 2 | |
| | 0 | 1 | | 0 | |

### Safety results

Safety analysis was performed on the safety population (n=105), and one patient assigned to placebo (n=106) was excluded from the ITT because the patient dropped out before treatment started. No treatment-related deaths were identified in the trial. Neither discontinuations nor dose reductions were considered as treatment related.

Events of any cause occurring during the observation period were comparable between the Nanvuranlat (85.7%) and placebo groups (82.9%). Grade 3 or higher adverse events occurred in 30.0% versus 22.9% of patients in the Nanvuranlat and placebo groups, respectively, and serious adverse events occurred in 21.4% versus 11.4% (Table 6). Treatment-related adverse events in the Nanvuranlat were 41.4%, which is less than in the placebo (57.1%). Grade 3 or higher treatment-related adverse events were reported in three patients treated with Nanvuranlat and included fatigue, decreased platelet counts, and hypertension.

**[Table 6]**

| Table 6: Comparison of adverse events (all grades, Grade 3 or more) between Nanvuranlat and placebo on safety endpoints | | | | |
|---|---|---|---|---|
| **Adverse Events** | **Nanvuranlat(n=70)** | | **Placebo(n=35)** | |
| | All grades | ≥ Grade 3 | All grades | ≥ Grade 3 |
| **At least one adverse event** | **60 (85.7%)** | **21 (30.0%)** | **29 (82.9%)** | **8 (22.9%)** |
| Abdominal discomfort | 2 (2.9%) | - | 2 (5.7%) | - |
| Constipation | 10 (14.3%) | - | 5 (14.3%) | - |
| Diarrhea | 8 (11.4%) | 1 (1.4%) | 3 (8.6%) | - |
| Nausea | 9 (12.9%) | - | 3 (8.6%) | - |
| Stomatitis | 4 (5.7%) | - | 2 (5.7%) | - |
| Vomiting | 5 (7.1%) | - | 4 (11.4%) | - |
| Fatigue | 1 (1.4%) | - | 4 (11.4%) | 1 (2.9%) |
| Malaise | 11 (15.7%) | 1 (1.4%) | 4 (11.4%) | - |
| Fever | 9 (12.9%) | - | 4 (11.4%) | - |
| Cholangitis | 10 (14.3%) | 9 (12.9%) | 3 (8.6%) | 2 (5.7%) |
| Nasopharyngitis | 1 (1.4%) | - | 2 (5.7%) | - |
| Blood creatinine elevation | 1 (1.4%) | - | 2 (5.7%) | - |
| Hypokalemia | | - | 2 (5.7%) | - |
| Hypophagia | 16 (22.9%) | - | 8 (22.9%) | 1 (2.9%) |
| Tumor pain | 1 (1.4%) | - | 2 (5.7%) | - |
| Cancer pain | 6 (8.6%) | 1 (1.4%) | 2 (5.7%) | - |
| Insomnia | 8 (11.4%) | - | 2 (5.7%) | - |
| Pruritus | 5 (7.1%) | - | 2 (5.7%) | - |
| Rash | 2 (2.9%) | - | 3 (8.6%) | - |
| High blood pressure | 5 (7.1%) | 2 (2.9%) | 1 (2.9%) | - |

All grades of adverse events were reported in 5.0% or more of patients.

### Consideration

This is the first randomized, controlled trial to demonstrate the clinical benefit of monotherapy with the LAT1 inhibitor Nanvuranlat compared to placebo. Significant differences in PFS between Nanvuranlat and placebo were observed in progressive patients refractory or intolerant to standard chemotherapy. The BTC patient population in this invention was much more advanced compared to the group of patients in which FOLFOX showed improvement as second-line therapy¹⁴. Eighty-three percent of patients were enrolled as third or subsequent line therapy (Table 1). Twenty-six percent of patients did not survive beyond 90 days. Due to this very advanced late BTC, the PFS Kaplan-Meier curve declined rapidly within 1.5 months in the placebo group (90% of patients reached event), which is consistent with observations from other trials in Japan¹⁵

Kaplan-Meier analysis of the LAT1 high-expressing subgroup showed that Nanvuranlat further improved PFS compared to the analysis in the overall patient population. Since high LAT1 expression has been documented as a poor prognostic factor^{6,7,16}, the efficacy of Nanvuranlat observed in patients with high LAT1 expression is supported. This is a surprising result considering that higher expression of the target VEGFR molecule results in higher ED50 values (i.e., lower drug efficacy) for VEGFR inhibitors¹⁷.

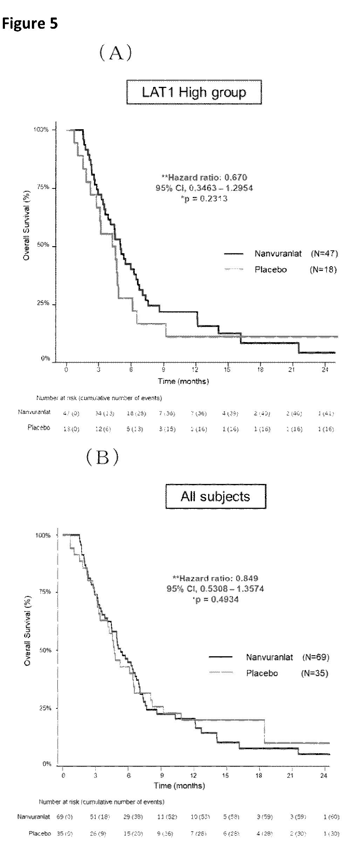

Although the OS hazard ratios were not statistically significant, Nanvuranlat showed an improvement in median OS compared to placebo, especially in the LAT1-high expressing patient population (Figures 5 and 6). The small number of patients and the fact that the patient population in this Phase II trial was too advanced and heavily pretreated are reasons why the improvement in OS was not statistically significant. Future trials are warranted to evaluate the efficacy of Nanvuranlat in early-stage patients and/or to identify additional factors, such as genetic mutations in BTC subtypes, to predict response to this potential treatment.

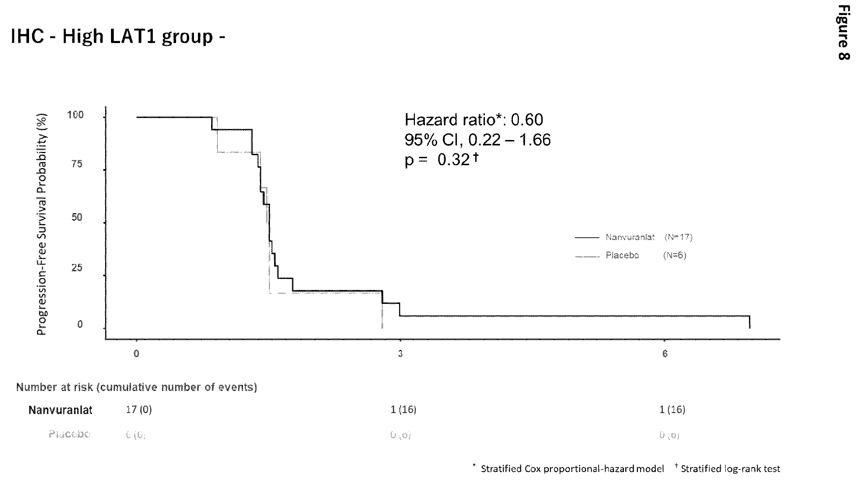

Kaplan-Meier analysis of the LAT1 high-expressing subgroup with IHC showed further improvement in PFS with Nanvuranlat compared to placebo (HR: 0.60, Cl95% 0.22-1.66, p=0.32; Figure 8).

With regard to the safety of Nanvuranlat, the incidence of AEs with Nanvuranlat was similar to that with placebo (Table 6). There were no deaths or discontinuations linked to treatment. Duration of treatment correlated well with the OS of Nanvuranlat, supporting its long-term use. This well-tolerated safety profile suggests that Nanvuranlat is suitable for the treatment of elderly but fragile BTC patients. This may be another clinical advantage of LAT1 inhibitors, as Nanvuranlat selectively acts on cancer cells but not on normal cells⁴.

Although all causal effects to treatment are excluded, the occurrence of cholangitis was notable in the Nanvuranlat group (Table 6). Adverse hepatic effects occurred in NAT2-rapid patients in an exploratory analysis of Phase I trial, which may involve the metabolism of Nanvuranlat¹¹,

In conclusion, Nanvuranlat monotherapy significantly improved PFS compared to placebo in patients with advanced and intractable BTC. Response to Nanvuranlat (PFS and OS) was enhanced in patients with higher LAT1 expression. Monotherapy with Nanvuranlat may be an option for late-stage patients with high LAT1 expression and EHC, GBC, or IHC subtypes, and may be considered in combination.

The following is a description of the upcoming Phase III trial using the biliary tract cancer treatment agent of the present invention.

### Phase III trial

Phase III multicenter, randomized, double-blind, placebo-controlled or external subject group-controlled trial to evaluate the efficacy and safety of Nanvuranlat in previously treated advanced BTC

### (1) Primary endpoint:

To compare the progression-free survival (PFS) overall survival (OS) of Nanvuranlat with placebo or external control group as assessed by blinded independent central review (BICR) in subjects with histologically or cytologically confirmed BTC, non-rapid NAT2 genotype, and high LAT1 score.

### (2) Secondary endpoint:

➢ Comparison among BTC subtypes (IHC, EHC, GBC)
➢ Comparison of patients with high and low LAT1 scores
➢ Comparison among NAT2 genotypes
➢ Comparison with placebo or external control group

### Test design

Treatment is continued until PD, unacceptable toxicity, or other discontinuation criteria are met. All subjects will be followed for survival at regular intervals for at least 24 months after discontinuation of trial therapy until death.

In each 14-day or fixed duration treatment cycle, subjects will receive either Nanvuranlat or placebo by intravenous infusion for 90 minutes or for a fixed duration, once daily, for 5 consecutive days or for a fixed number of days. Thereafter, there will be a 9-day or constant period of no treatment, and subjects will continue treatment for 14-day or constant period cycles until PD, unacceptable toxicity, or other discontinuation criteria are met. Radiographic imaging for tumor lesion measurement and disease assessment will be performed after 3 cycles (i.e., 6 weeks), but may be performed off schedule at the discretion of the principal investigator.

### References

1.Nagino M, Hirano S, Yoshitomi H, et al. Clinical practice guidelines for the management of biliary tract cancers 2019: The 3rd English edition. J Hepatobiliary Pancreat Sci. 2021;28:26-54.
2.Banales JM, Marin JJG, Lamarca A, et al. Biliary tract cancer 2020: the next horizon in mechanisms and management. Nat Rev Gastroenterol Hepatol 2020;17:557-588.
3.Sato M, Harada-Shoji N, Toyohara T et, al., L-type amino acid transporter 1 is associated with chemoresistance in breast cancer via the promotion of amino acid metabolism. Sci Rep 2021;11:589.
4.Kanai Y, Amino acid transporter LAT1 (SLC7A5) as a molecular target for cancer diagnosis and therapeutics. Pharmacol Ther 2022;230:107964.
5.Yanagida, O, Kanai Y, Chairoungduaet A, et al. Human L-type amino acid transporter 1 (LAT1): characterization of function and expression in tumor cell lines. Biochim Biophys Acta 2001;1514 291-302.
6.Kaira K, Sunose Y, Ohshima Y, et al. Clinical significance of L-type amino acid transporter 1 expression as a prognostic marker and potential of new targeting therapy in biliary tract cancer. BMC Cancer 2013;13:482.
7.Yanagisawa N, Hana K, Nakada N, et al. High expression of L-type amino acid transporter 1 as a prognosis marker in bile duct adrenocarcinomas. Cancer Med 2014;3:1246-1255.
8.Yothaisong S, Dokduang H, Anzai N, et al. Inhibition of L-type amino acid transporter I activity as a new therapeutic target for biliary tract cancer treatment. Tumor Biology;39:1010428317694545.
9.Okanishi H, Ohgaki R, Okuda S, et al. Proteomics and phosphoproteomics reveal key regulators associated with cytostatic effect of amino acid transporter. Cancer Science 2021;112:871-883.
10.Nishikubo K, Ohgaki R, Okanishi H, et al. Pharmacologic inhibition of LAT1 predominantly suppresses transport of large neutral aminoacids and downregulates global translation in cancer cells. J Cell Mol Med 2022;26:5246-5256.
11.Okano N, Naruge D, Kawai K, et al. First-in-human phase I study of JPH203, an L-type amino acid transporter 1 inhibitor, in patients with advanced solid tumors. Invest New Drugs 2020;38:1495-1506.
12.Toyoshima J, Kusuhara H, Wempe MF, et al. Investigation of the Role of Transporters on the Hepatic Elimination of an LAT1 Selective Inhibitor JPH203. J Pharm Sci 2013;102:3228-38.
13.Hein DW, Doll MA, Accuracy of various human NAT2 SNP genotyping panels to infer rapid, intermediate and slow acetylator phenotypes. Pharmacogenomics 2012; 13(1):31-41.
14.Lamarca A, Palmer DH, Wasan HS, et al., Second-line FOLFOX chemotherapy versus active symptom control for advanced biliary tract cancer (ABC-06): a phase 3, open-label, randomised, controlled trial. Lancet Oncology 2021;22 690-701.
15.Ueno M, et al, Ikeda M, Morizane C, et al., Nivolumab alone or in combination with cisplatin plus gemcitabine in Japanese patients with unresectable or recurrent biliary tract cancer: a non-randomised, multicentre, open-label, phase 1 study. Lancet Gastroenterol Hepatol. 2019;4:611-621.
16.*Rina Otani, Hidehiko Takigawa, Ryo Yuge et at, Cancers,2023,15:1383.*
17.I K Dev, R E Dornsife, T M Hopper, et al, British Journal of Cancer(2004) 91,1391-1398*.*

### Industrial applicability

The present invention may provide a biliary tract cancer treatment agent that is effective against biliary tract cancer, which has been difficult to treat effectively in the past. The present invention may also provide a biliary tract cancer treatment agent that is effective against BTC, especially EHC and GBC among BTC, in patients with a specific NAT2 genotype, especially the Non-Rapid (Slow and/or Intermediate) type of NAT2 genotype.

### Brief description of the drawing

[Figure 1] This figure shows the background of biliary tract cancer patients assigned to the FAS population in this trial.
[Figure 2] Forest plot of PFS hazard ratios by BICR for the major subgroups.
[Figure 3] This figure shows all adverse events of Grade 3 or higher and SAE in the safety endpoints.
[Figure 4] (A) shows the primary PFS with BICR in the LAT1 high-expressing group, and (B) shows the primary PFS with BICR in all patient populations with FAS.
[Figure 5] (A) shows overall survival (OS) in the LAT1 high-expressing group, and (B) shows overall survival (OS) in all patient populations with FAS.
[Figure 6] Correlation between duration of treatment and overall survival in the LAT1 high-expressing group.
[Figure 7] Kaplan-Meier plot of PFS between Nanvuranlat and placebo in a patient population with GBC/EHC.
[Figure 8] Kaplan-Meier plot of PFS between Nanvuranlat and placebo in a patient population with IHC and in the LAT1 high-expressing group.

## Claims

1. A treatment agent for advanced biliary tract cancer that is refractory or intolerant of standard chemotherapy in a subject, comprising a LAT1 inhibitor.

2. The treatment agent according to Claim 1, wherein the LAT1 inhibitor is O-(5-amino-2-phenylbenzoxazol-7-yl)methyl-3 ,5-dichloro-L-tyrosine, or a pharmaceutically acceptable salt thereof.

3. The treatment agent according to Claim 1, wherein the advanced biliary tract cancer in the subject that is refractory or intolerant of standard chemotherapy is selected from the group consisting of extrahepatic biliary tract cancer and gall bladder cancer.

4. The treatment agent according to Claim 3, wherein the biliary tract cancer is extrahepatic biliary tract cancer.

5. The treatment agent according to Claim 3, wherein the biliary tract cancer is gall bladder cancer.

6. The treatment agent according to Claim 3, wherein the subject is a patient with high LAT1 expression.

7. The treatment agent according to Claim 3 or 6, wherein the subject is a subject identified as having a Non-Rapid (Slow and/or Intermediate) type NAT2 gene.

8. The treatment agent according to Claim 1, wherein the subject is a patient with high LAT1 expression.

9. The treatment agent according to Claim 8, wherein the biliary tract cancer is extrahepatic biliary tract cancer or gall bladder cancer.

10. The treatment agent according to Claim 9, wherein the subject is a subject identified as having a Non-Rapid (Slow and/or Intermediate) type NAT2 gene.

11. The treatment agent according to Claim 1, wherein the biliary tract cancer is intrahepatic biliary tract cancer.

12. The treatment agent according to Claim 11, wherein the subject is a patient with high LAT1 expression.

13. The treatment agent according to Claim 12, wherein the subject is a subject identified as having a Non-Rapid (Slow and/or Intermediate) type NAT2 gene.

14. The treatment agent according to any one of Claims 1-3, 8 and 11, wherein 1-60 mg/m² of LAT1 inhibitor is administered to the subject.

15. The treatment agent according to Claim 14, wherein 12.5 to 60 mg/m² of LAT1 inhibitor is administered to the subject.

16. The treatment agent according to Claim 15, wherein 12.5 to 25 mg/m² of LAT1 inhibitor is administered to the subject.

17. The treatment agent according to any one of Claims 1-3, 8 and 11, wherein a fixed amount of the LAT1 inhibitor to be administered to the subject is administered intravenously continuously over a fixed time period.

18. The treatment agent according to Claim 17, wherein 100 mL of the LAT1 inhibitor is administered intravenously over 90 minutes.

19. The treatment agent according to any one of Claims 1-3, 8 and 11, wherein the subject is a human.

20. A pharmaceutical composition comprising a biliary tract cancer treatment agent according to Claim 1, and a pharmaceutically acceptable additive.

21. A treatment agent for of curatively unresectable biliary tract cancer that has become refractory to cancer chemotherapy and has worsened in a subject, comprising a LAT1 inhibitor.

22. The treatment agent according to Claim 21, wherein the LAT1 inhibitor is O-(5-amino-2-phenylbenzoxazol-7-yl)methyl-3,5-dichloro-L-tyrosine, or a pharmaceutically acceptable salt thereof.

23. The treatment agent according to Claim 21, wherein the biliary tract cancer is selected from the group consisting of extrahepatic biliary tract cancer and gall bladder cancer.

24. The treatment agent according to Claim 23, wherein the biliary tract cancer is extrahepatic biliary tract cancer.

25. The treatment agent according to Claim 23, wherein the biliary tract cancer is gall bladder cancer.

26. The treatment agent according to Claim 23, wherein the subject is a patient with high LAT1 expression.

27. The treatment agent according to Claim 23 or 26, wherein the subject is a LAT1-high expressing patient with Non-Rapid N-acetyltransferase 2.

28. The treatment agent according to Claim 21, wherein the subject is a patient with high LAT1 expression.

29. The treatment agent according to Claim 28, wherein the biliary tract cancer is extrahepatic biliary tract cancer or gall bladder cancer.

30. The treatment agent according to Claim 29, wherein the Non-Rapid type is Slow and/or Intermediate.

31. The treatment agent according to Claim 21, wherein the biliary tract cancer is intrahepatic biliary tract cancer.

32. The treatment agent according to Claim 31, wherein the subject is a patient with high LAT1 expression.

33. The treatment agent according to Claim 32, wherein the subject is a LAT1-high expressing patient with Non-Rapid N-acetyltransferase 2.

34. The treatment agent according to any one of Claims 21-23 and 28 and 31, wherein 1-60 mg/m² of LAT1 inhibitor is administered to the subject.

35. The treatment agent according to Claim 34, wherein 12.5 to 60 mg/m² of LAT1 inhibitor is administered to the subject.

36. The treatment agent according to Claim 35, wherein 12.5 to 25 mg/m² of LAT1 inhibitor is administered to the subject.

37. The treatment agent according to any one of Claims 21-23 and 28 and 31, wherein a fixed amount of the LAT1 inhibitor to be administered to the subject is administered intravenously continuously over a fixed time period.

38. The treatment agent according to Claim 37, wherein 100 mL of the LAT1 inhibitor is administered intravenously over 90 minutes.

39. The treatment agent according to any one of Claims 21-23 and 28 and 31, wherein the subject is a human.

40. A pharmaceutical composition comprising the treatment agent according to Claim 21, and a pharmaceutically acceptable additive.
